# EUROPEAN PATENT SPECIFICATION

(11) **EP 3 019 016 B1**
(45) Date of publication and mention of the grant of the patent: **27.12.2017**
(21) Application number: 14734442.8
(22) Date of filing: 26.06.2014
(51) Int. Cl.: A01N 59/12, C11D 3/48, C11D 1/04, C11D 1/12, C11D 1/66, C11D 1/38, A61Q 17/00, A61K 8/20, C11D 1/14, C11D 1/22

(54) **DETERGENT COMPOSITION**
REINIGUNGSMITTELZUSAMMENSETZUNG
COMPOSITION DE DÉTERGENT

(30) Priority: 11.07.2013 EP 13176061
(43) Date of publication of application: 18.05.2016
(73) Proprietor: Unilever N.V., 3013 AL Rotterdam (NL); Unilever PLC, London, Greater London EC4Y 0DY (GB)
(72) Inventor: BISWAS, Sarmistha, Whitefield Bangalore 560 066 (IN); DASGUPTA, Anindya, Whitefield Bangalore 560 066 (IN); NETHAJI, Alagirisamy, Whitefield Bangalore 560 066 (IN); SAJI, Maya Treesa, Whitefield Bangalore 560 066 (IN); SHRESTH, Rudra Saurabh, Whitefield Bangalore 560 066 (IN)
(74) Representative: Reijns, Tiemen Geert Pieter
(86) International application number: PCT/EP2014/063470
(87) International publication number: WO 2015/003912

(56) References cited:
- WO-A1-00/18867
- WO-A1-00/57703
- WO-A1-01/11969
- WO-A1-85/04184
- WO-A1-97/07202
- US-A- 3 338 837
- US-A- 3 821 124

## Description

### Field of the invention

The invention relates to cleaning compositions having anti-microbial benefits, in particular cleaning compositions having enhanced anti-microbial benefit because of the synergistic interaction between a sulphonated anionic surfactant and an iodine containing inorganic salt selected from potassium iodide and potassium iodate.

### Background of the invention

Present day consumers appreciate detergent compositions for amongst others laundry cleaning and conditioning as well as household cleaning purposes; including hard surface cleaning and treatment composition, as well as dishwashing compositions. Several anti-microbial agents and compositions are known in the art, but many of them contain halogens, halogen oxidants or halogen salts in high amounts that can be harmful to humans

There is a long felt need for detergent compositions that have low levels of halogens, halogen oxidants or halogen salts and based on harmless and environmentally safe anti-microbial substances.

WO 2001/38626 discloses stain treatment compositions comprising surfactant and iodate at acidic pH. The iodate acts as an oxidising agent and gets reduced to iodine under acidic conditions. However acidic pH is not suitable for general detergent compositions, since stains and soil are better removed at alkaline pH. In WO2001/38626 it has been attempted to overcome the drawback of a low pH by adding bleach to the composition in a separate sub composition. This is undesirable for processing and packaging and needlessly increases the cost of the product. Additionally, bleach may cause damage to coloured fabrics and surfaces.

WO2008/137769 discloses warewashing compositions including a hardness ion (e. g. , magnesium and calcium ions) as a corrosion inhibitor. Such compositions can be used to reduce corrosion or etching of glass, porcelain and ceramic, it also relates to methods employing these warewashing compositions. WO2008/137769 particularly discloses a warewashing composition including a cleaning agent having a detersive amount of a surfactant, an alkaline source in an amount effective to provide a use composition having a pH of at least about 8 and a corrosion inhibitor in an amount sufficient for reducing corrosion of glass. The corrosion inhibitor as mentions includes a salt of calcium and/or magnesium and iodide salts thereof are disclosed. However no mention is made to anti-microbial effect.

Similarly, US 2,599,140 discloses an iodine detergent for cleansing hands comprising a solvent mixture of a polyalkylene glycol and glycerine having in solution therein elemental iodine, an alkali metal iodide and a detergent compatible with the other ingredients, which detergent is an anionic sulphated synthetic detergent. However, this composition is slightly acidic and remains so on dilution and this is desirable as the iodine is more stable in slightly acidic composition and eliminates the risk of inactivation of the iodine with reaction with an alkali. Therefore, this composition would be undesirable in laundry and there still remains a need to provide an anti-microbial composition having neutral to alkaline pH with low levels of halogens, halogen oxidants or halogen salts that can be used with a variety of surfactants, including sulphonated anionic surfactants.

US 3,821,124 discloses detergent and shampoo compositions comprising an anionic, non-soap, non-sulphonate surfactant and iodate. However, sulphonated surfactants are especially preferred in many detergent compositions due to their low cost and high activity. It therefore remains to be desired to provide an anti-microbial composition having neutral to alkaline pH, that can be used with a variety of surfactants, including sulphonated and soap based anionic surfactants.

Similarly, US 3,338,837 relates to a highly germicidally-active iodinated detergent composition of high concentration comprising 4 to 15% of an organic detergent which may be anionic, cationic or non-ionic detergent, 10 to 30% of an organic halogen oxidant, 0.5 to 5% of an inorganic iodide and an inorganic alkalizer sufficient to raise the pH of the composition to between 7.8 to 8.4. This composition comprises a high amount of halogen oxidant which is not preferred in the present invention. It therefore remains to be desired to provide anti-microbial compositions that have low levels of halogens, halogen oxidants or halogen salts.

Detergent compositions having a neutral to alkaline pH and that have low levels of halogens, halogen oxidants, or halogen salts providing good cleaning and improved anti-microbial benefits remain to be desired.

It is therefore an object of the present invention to provide a detergent composition with good cleaning and enhanced anti-microbial benefit at neutral to alkaline pH.

It is another object of the present invention to provide an anti-microbial composition having low levels of halogens, halogen oxidants or halogen salts.

Surprisingly, it has been found that at a neutral to alkaline pH, a synergistic anti-microbial activity may be obtained between a sulphonated anionic surfactant and 0.01 to. 0.3%w of an iodine containing inorganic salt selected from potassium iodate or potassium iodide.

### Summary of the invention

Accordingly, in a first aspect, the present invention provides a detergent composition having anti-microbial benefits comprising 0.5% to 90%w of a sulphonated anionic surfactant and 0.01-0.3%w of an iodine containing inorganic salt selected from potassium iodate and potassium iodide, wherein the pH of the composition is between 7 and 12.

In a second aspect, the invention provides a wash liquor comprising the composition according to the invention, wherein the iodine containing inorganic salt is in a concentration of between 1 ppm to 100ppm.

In a third aspect, the invention provides an antimicrobial wash liquor, wherein the composition according to the invention is dosed in a concentration of between 100ppm and 20000ppm in the wash liquor.

In a fourth aspect, the invention provides a packaged cleaning composition, wherein the composition according to the invention is diluted with water in a ratio of between 1:10 to 1:1000 in a direct application container.

In a fifth aspect, the invention provides a method for rendering a substrate anti-microbial comprising the steps in sequence of preparing a 0.05% - 1 % by weight solution of the composition according to the invention in water, allowing the substrate to be in contact with the solution for at least 1 min and; drying the substrate.

In the context of the present invention, the reference to "microbial" in the term anti-microbial includes bacteria, archaea, viruses, protozoa, fungi, algae or cysts.

In the context of the present invention, the reference to "substrate" typically means fabric and household surfaces.

By low levels it is meant that the composition does not contain more than 0.5%w of halogens, halogen oxidants or halogen salts.

In the context of the invention, the most preferred applications are laundry cleaning, laundry conditioning, and household cleaning while the present invention could be applicable in other fields like personal care as well.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

### Detailed description of the invention

In a first aspect, the invention relates to a detergent composition comprising a sulphonated anionic surfactant and an iodine containing inorganic salt selected from potassium iodide and potassium iodate, wherein the pH of the composition is between 7 and 12.

Without wishing to be bound by a particular theory, the combination of specific surfactants with specific iodine salts provides an anti microbial effect.

### Surfactant

The surfactant in the composition is sulphonated anionic surfactants.

In general, these surfactants are described in well known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981.

Anionic surfactants are the most preferred in laundry detergent compositions. Anionic surfactants for the detergent compositions which are used in the present invention are sulphonated anionic surfactants. Suitable sulphonated anionic surfactants include (usually) water-soluble alkali metal salts of organic sulphonates having alkyl radicals typically containing from about 8 to about 22 carbon atoms (the term alkyl being used to include the alkyl portion of higher acyl radicals), alkaryl sulphonates, alkanoyl isethionates, alkyl sulphosuccinates, N-alkoyl sarcosinates, linear alkyl benzene sulfonate (LAS) and alpha-olefin sulphonates. The alkyl and acyl groups contain from 8 to 22 carbon atoms, preferably 8 to 18 carbon atoms, still more preferably 12 to 15 carbon atoms and may be unsaturated.

Examples of suitable anionics include linear alkyl benzene sulfonate (LAS), Methyl Ester Sulphonate (MES), ammonium lauryl sulphosuccinate, sodium cocoyl isethionate, sodium lauroyl isethionate, and sodium N-lauryl sarcosinate.

The surfactant is present in the composition in a concentration of between 0.5% and 90% by weight, preferably at least 1%, more preferably at least 2%, still more preferably at least 3%, even more preferably at least 5% or at least 9% or even at least 15% by weight, but typically not more than 60%, still more preferably not more than 40% or even not more than 30% by weight of the composition.

The surfactant is present in the wash liquor in a concentration of between 50ppm and 2000ppm, preferably at least 100ppm, more preferably at least 150ppm, but typically not more than 1500ppm or even not more than 1000ppm.

### Iodine containing inorganic salt

The iodine containing inorganic salt according to the present invention is selected from potassium iodide and potassium iodate.

The iodine containing inorganic salt is present in the composition in a concentration of 0.01 to 0.3% by weight of the composition.

The iodine containing inorganic salt is present in the wash liquor in a concentration of between 1 ppm and 100ppm, preferably between 1 ppm and 50ppm, more preferably between 1 ppm and 35ppm.

### pH of the composition

Anti-microbial activity of iodine salts is generally seen in an acidic pH. Therefore, iodine salt containing compositions having an acidic pH are commonly used as anti-microbial compositions. This is because antimicrobial agents such as iodine are more effective in acidic pH.

In the present invention, surprisingly the combination of a surfactant and the iodine containing inorganic salts has been found to have a synergistic antimicrobial activity at neutral to alkaline pH making it suitable for cleaning as well.

Therefore, the pH of the composition of the present invention is between 7 and 12. The best results are obtained when the pH of the composition is less than 11.

### Other ingredients

The composition according to the invention may further comprise conventional ingredients including builder, electrolyte and/or fillers. When one or more of these ingredients are present, together they are typically present in the composition in a concentration of between 9% and 94.99% by weight of the total composition.

The composition may further comprise hydrotropes, soil release polymers, perfume, fluorescers, shading dyes, sequestrants, and anti re-deposition agents.

### Method of use

For laundry main wash including hand wash or conditioning application of the composition of the invention, the composition according to the invention is preferably dosed to a concentration of between 100ppm and 20000ppm, preferably between 300ppm and 10000ppm, more preferably between 1000ppm and 8000ppm in the wash liquor.

For horizontal axis washing machines the dosing is preferably between 100ppm and 10000ppm, more preferably not less than 500ppm, or even not less than 1000ppm, but typically not more than 5000ppm in the wash liquor.

For vertical axis washing machines the dosing is preferably between 100ppm and 15000ppm, more preferably not less than 500ppm, or even not less than 1000ppm, but typically not more than 10000ppm in the wash liquor.

### Product format

The composition may be used neat or diluted.

For laundry composition the composition is added to the washing process neat and diluted to the concentration as indicated above. The composition for laundry use is preferably a liquid or a powder. However, tablets, bars, gels and pastes are also contemplated in the context of the present invention. A typical dosage for a detergent composition to a washing process is 1-20 grams of detergent composition per litre of wash liquor for horizontal axis washing machines and hand washing process, while the dosage to a vertical axis washing machine is generally in the order of 0.1 to 3 grams per litre of wash liquor.

For hard surface cleaning products the composition is typically applied in a diluted form, directly to the surface. Such a composition is preferably diluted with water in a ratio of between 1:10 to 1:1000. Ideally the composition is packaged in a direct application container, such as a trigger spray dispenser.

For dishwashing purposes the composition according to the invention is applied neat. The composition according to the invention for dishwashing use is preferably a liquid, a paste, a powder or a shaped composition. Both manual dishwashing and machine dishwashing are considered in the context of the present invention.

### A method for rendering a substrate anti-microbial

A method for rendering a substrate anti-microbial comprising the steps in sequence of preparing a 0.05% - 1% by weight solution of the composition according to the invention in water, allowing the substrate to be in contact with the solution for at least 1 minute and drying the substrate.

The contact time required is dependent on the target microorganism. An enhanced antibacterial effect is achieved with a contact time of at least 1 minute for bacteria but in the case of viruses, a contact time of at least 5 minutes is required depending on the type and structure of the virus.

The invention will now be illustrated by means of the following non-limiting examples.

### Examples

### Materials

### Iodine containing inorganic salt:

Potassium Iodide, Potassium Iodate (ex Sigma Aldrich)

**Surfactants:**

| | |
|---|---|
| Suphonated Anionic: | Sodium salt of dodecyl benzene sulphonate (DOBS) commonly referred to as linear alkyl sulphonate (LAS) (ex Rhodia Speciality Chemicals, Sigma Aldrich) |
| Sulphated Anionic: | Sodium lauryl Ether Sulphate(SLES)(ex Galaxy Surfactants) |
| Cationic: | Benzalkonium chloride (BAC), (ex Sigma Aldrich Cas No. 63449-41-2) |
| Non-Ionic: | Linear Lauryl Alcohol Ethoxylate(EO₅)(ex Galaxy Surfactants) |

### Method

### Bacteria:

The anti-microbial effect of the composition according to the invention was demonstrated in by a bacterial plate test.

### Test solutions

Different test solution of surfactant and the iodine salt in water were prepared in the indicated concentrations. The test solutions as prepared had a pH of 7. Some experiments were done with the composition at pH 7, while for others the pH was adjusted to 10.5. The pH was adjusted with sodium carbonate solution (0.5 g/L Na₂CO₃ in water) or with sodium hydroxide.
*Protocol: BS EN 1040 (modified with specific contact time)*
*Test bacteria: S.aureus* ATCC 6538
   *E.hirae* ATCC 10541

The test bacteria was grown overnight at 37°C on TSA plate. The grown culture colonies were resuspended in 0.8% saline solution. The culture cell density was adjusted to get the final count of 1*10⁸ cfu/ml, based on a 620nm optical density calibration chart (0.8 OD at 620nm). 9ml of the test solution was taken in a sterile sample container and 1 ml of the test culture was added. After the specified contact time, 1 ml of the above mixture was immediately neutralized in 9 ml D/E broth as commonly used in the art. This was again serially diluted in D/E broth and plated on TSA (a.k.a. Tryptic Soy Agar; commonly used in the art) in duplicates. In case of the control, 1 ml of test culture was added to 9 ml of saline and was serially diluted and plated on TSA. After solidification, the plates were incubated at 37°C for 48 hrs and the residual colonies were counted. All the antimicrobial experiments were performed under aseptic condition under laminar air flow and all the agar media and D/E dilution tubes were autoclaved (15 psig, 121 deg C, 15-20 min) before use.

In the examples below the log values of the residual colony forming units (cfu's) is given and compared. In comparing log value 1 point more reduction mean a 10 fold higher kill. So for instance when under comparative conditions the residual log value is 4 (i.e. 10000 cfu) and the inventive composition it is 2 (i.e. 100). That means that the end culture that is treated with the inventive composition has only 1% of the residual bacteria as the comparative composition. So 2 points on the log scale makes a big difference when it comes to hygiene.

### Virus:

A standard EST protocol was (EN 14476) was followed for the evaluation of activity of antiviral actives in suspension test. Antiviral activity of actives was tested in clean conditions.

Clean conditions: To create clean conditions, 0.3g of bovine albumin fraction V was dissolved in 100 ml of water and sterilised by passing the solution through a 0.2 µm filter.

Preparation of test mixture: To prepare the test mixture, 1 ml of interfering substance (Bovine Albumin) was pipetted into a suitable container, followed by addition of 1 ml of test virus suspension. To this, 8ml of product test solution was added. At the end of chosen contact time period, 100µl of the test mixture was added to resin column. Centrifugation of microspin column (735 xg, 2 min). The elutant was diluted up to 10⁻⁶ in ice cold virus growth medium the infectivity of each dilution was measured by TCID₅₀ using Spaerman-Karber formula.

Titration of Virus: Monolayers of cells were grown in 96-well tissue-culture plates (Costar) at 37°C in an atmosphere of 95% air and 5% carbon dioxide (CO₂). Tenfold dilutions of virus suspension were prepared in virus growth medium. One hundred microlitres of each dilution was added to five replicate wells in 96-well tissue culture plates. Cell controls were included on each plate. Plates were incubated at 37°C in an atmosphere of 95% air/ 5% CO_{2.} Cultures were observed daily for viral cytopathic effect for a period of seven days after which they were discarded. The virus titre was calculated by TCID₅₀ (Tissue culture infective dose) using the Spaerman-Karber formula.

### The formula is:

Negative logarithm of the 50% end point = Negative logarithm of the highest virus concentration used - [(Sum of % affected at each dilution /100-0.5)* (Ig of dilutions)]

### Example 1: Anti-microbial effect of Potassium Iodide and sulphonated Anionic surfactant (Sodium LAS) on S.aureus at pH 7

In this example the improved anti-microbial efficacy of iodide (KI, potassium iodide) and a sulphonated anionic surfactant (LAS, Sodium linear alkylbenzene sulphonate) is demonstrated at pH 7 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 15ppm KI | 1.20E+07 | 7.1 | 0.1 |
| 100ppm LAS | 1.40E+06 | 6.1 | 1.1 |
| 300ppm LAS | 9.00E+04 | 5.0 | 2.2 |
| 15ppm KI + 100ppm LAS | 1.70E+03 | 3.2 | 4.0 |
| 15ppm KI + 300ppm LAS | 1.10E+03 | 3.0 | 4.2 |

As shown in the table above, the individual anti-microbial activity of KI and LAS alone is limited, while the combined efficacy is at least 2-log higher (i.e. 100 fold more reduction). The combined efficacy does not substantially increase further with increase in the concentration of LAS at pH 7, but the kill is still a 100 fold more than without the KI and 10000 fold more than without LAS.

### Example 2: Anti-microbial effect of Potassium Iodate and sulphonated Anionic surfactant (Sodium LAS) on S.aureus at pH 7

In this example the improved anti-microbial efficacy of iodate (KIO₃, potassium iodate) and a sulphonated anionic surfactant (LAS, Sodium linear alkylbenzene sulphonate) is demonstrated at pH 7 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 15ppm KIO₃ | 1.20E+07 | 7.1 | 0.1 |
| 100ppm LAS | 1.40E+06 | 6.1 | 1.1 |
| 300ppm LAS | 9.00E+04 | 5.0 | 2.2 |
| 15ppm KIO₃+ 100ppm LAS | 2.80E+03 | 3.4 | 3.8 |
| 15ppm KIO₃+ 300ppm LAS | 1.70E+03 | 3.2 | 4.0 |

As shown in the table above, the individual anti-microbial activity of KIO3 and LAS alone is limited, while the combined efficacy is about 2-log higher (i.e. 100 fold more reduction).

### Example 3: Anti-microbial effect of Potassium Iodide and sulphonated Anionic surfactant (Sodium LAS) on S.aureus at pH 10.5

In this example the improved anti-microbial efficacy of iodide (KI, potassium iodide) and a sulphonated anionic surfactant (LAS, Sodium linear alkylbenzene sulphonate) is demonstrated at pH 10.5 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 15ppm KI | 1.20E+07 | 7.1 | 0.1 |
| 100ppm LAS | 2.00E+06 | 6.3 | 0.9 |
| 300ppm LAS | 1.20E+05 | 5.1 | 2.1 |
| 15ppm KI +100ppm LAS | 1.00E+05 | 5.0 | 2.2 |
| 15ppm KI +300ppm LAS | 1.00E+04 | 4.0 | 3.2 |

As shown in the table above, the individual anti-microbial activity of KI and LAS alone is limited, while the combined efficacy is at least 1-log higher (i.e. 10 fold more reduction). The combined efficacy increases with increase in the concentration of LAS at pH 10.5.

### Example 4: Anti-microbial effect of Potassium Iodate and sulphonated Anionic surfactant (Sodium LAS) on S.aureus at pH 10.5

In this example the improved anti-microbial efficacy of iodate (KIO₃, potassium iodate) and a sulphonated anionic surfactant (LAS, Sodium linear alkylbenzene sulphonate) is demonstrated at pH 10.5 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 15ppm KIO₃ | 1.20E+07 | 7.1 | 0.1 |
| 100ppm LAS | 2.00E+06 | 6.3 | 0.9 |
| 300ppm LAS | 1.20E+05 | 5.1 | 2.1 |
| 15ppm KIO₃+100ppm LAS | 1.80E+05 | 5.3 | 1.9 |
| 15ppm KIO₃+300ppm LAS | 1.50E+04 | 4.2 | 3.0 |

As shown in the table above, the individual anti-microbial activity of KIO3 and LAS alone is limited, while the combined efficacy is about 1-log higher (i.e. 10 fold more reduction). The combined efficacy increases further with increase in the concentration of LAS at pH 10.5.

### Comparative Example 5: Anti-microbial effect of Potassium Iodide and Cationic surfactant (BKC) on S.aureus at pH 7

In this example the improved anti-microbial efficacy of iodide (KI, potassium iodide) and a cationic surfactant (BKC, Benzalkonium chloride) is demonstrated at pH 7 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 40ppm BKC | 9.90E+05 | 6.0 | 1.2 |
| 15 ppm KI | 1.30E+07 | 7.1 | 0.1 |
| 15 ppm KI + 40ppm BKC | 1.10E+05 | 5.0 | 2.2 |
| 100 ppm BKC | 0.00E+00 | | 7.2 |
| 100 ppm BKC + 15 ppm KI | D.OOE+00 | | 7.2 |

As shown in the table above, the individual anti-microbial activity of KI and BKC alone is limited, while the combined efficacy is about 1-log higher (i.e. 10 fold more reduction).

### Comparative Example 6: Anti-microbial effect of Potassium Iodate and Cationic surfactant (BKC) on S.aureus at pH 7

In this example the improved anti-microbial efficacy of iodate (KIO₃, potassium iodate) and a cationic surfactant (BKC, Benzalkonium chloride) is demonstrated at pH 7 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 40ppm BKC | 9.90E+05 | 6.0 | 1.2 |
| 15 ppm KIO3 | 1.30E+07 | 7.1 | 0.1 |
| 15ppm KIO3 + 40ppm BKC | 1.00E+05 | 5.0 | 2.2 |
| 100 ppm BKC | 0.00E+00 | | 7.2 |
| 100ppm BKC+15ppm KIO3 | 0.00E+00 | | 7.2 |

As shown in the table above, the individual anti-microbial activity of KIO3 and BKC alone is limited, while the combined efficacy is about 1-log higher (i.e. 10 fold more reduction).

### Comparative Example 7: Anti-microbial effect of Potassium Iodide and sulphated Anionic surfactant (SLES) on E.hirae at pH 7

In this example the improved anti-microbial efficacy of iodide (KI, potassium iodide) and a sulphated anionic surfactant (SLES, Sodium lauryl ether sulfate) is demonstrated on a different bacteria, *E.hirae* at pH 7 and compared to the individual components as well as the untreated control sample. The contact time was 1 minute.

| | Residual cfu/ml | Residual log (cfu/ml) | Log reduction |
|---|---|---|---|
| *E.hirae* control | 9.00E+07 | 8.0 | |
| 100 ppm SLES | 6.50E+07 | 7.8 | 0.2 |
| 15 ppm KI | 8.50E+07 | 7.9 | 0.1 |
| 100ppm SLES + 15ppm KI | 1.50E+07 | 7.2 | 0.8 |

As shown in the table above, the combined efficacy of KI and SLES is only about 0.5 log higher which is not preferred.

### Comparative Example 8: Anti-microbial effect of Potassium Iodate and sulphated anionic surfactant (SLES) on E.hirae at pH 7

In this example the improved anti-microbial efficacy of iodate (KIO₃, potassium iodate) and an anionic surfactant (SLES, Sodium lauryl ether sulfate) is demonstrated on a different bacteria, *E.hirae* at pH 7 and compared to the individual components as well as the untreated control sample. The contact time was 1 minute.

| | Residual cfu/ml | Residual log (cfu/ml) | Log reduction |
|---|---|---|---|
| *E.hirae* control | 9.00E+07 | 8.0 | |
| 100 ppm SLES | 6.50E+07 | 7.8 | 0.2 |
| 15 ppm KIO3 | 9.00E+07 | 8.0 | 0.0 |
| 100ppm SLES +15ppm KIO3 | 1.50E+07 | 7.2 | 0.8 |

As shown in the table above, the combined efficacy of KIO3 and SLES is only about 0.5 log higher which is not preferred.

### Comparative Example 9: Anti-microbial effect of Potassium Iodide and Non-ionic surfactants (EO5) on S.aureus at pH 7

In this example, the improved anti-microbial efficacy of iodide (KI, potassium iodide) and non-ionic surfactants (EO5) is demonstrated at pH 7 and compared to the individual components as well as the untreated control sample. The contact time was 1 minute.

| | Residual cfu/ml | Residual log (cfu/ml) | Log reduction |
|---|---|---|---|
| *S.aureus* control | 1.70E+07 | 7.2 | |
| 20 ppm KI | 1.60E+07 | 7.2 | 0.0 |
| 100ppm EO5 | 1.70E+07 | 7.2 | 0.0 |
| 100ppm EO5 + 20ppm KI | 8.50E+06 | 6.9 | 0.3 |

As shown in the table above, the individual anti-microbial activity of KI and the non-ionic surfactants (EO5) alone is limited, while the combined efficacy is at least 0.3-log higher. Even though non-ionic surfactants are less preferred in the present invention, there still exists a synergistic effect in the combination of KI and the non-ionic surfactants.

### Example 10: Effect of increased concentration of surfactant on antimicrobial activity at pH 7.3

In this example, the concentration of the surfactant is increased substantially to check if there is an improved anti-microbial efficacy at high levels of the surfactant alone. The contact time was 1 minute.

| | Residual cfu/ml | Residual log (cfu/ml) | Log reduction |
|---|---|---|---|
| *S.aureus* control | 1.60E+07 | 7.2 | |
| 15ppm KI | 1.50E+07 | 7.2 | 0.0 |
| 750 ppm NaLAS | 1.00E+05 | 5.0 | 2.2 |
| 1500ppm NaLAS | 8.60E+0₄ | 4.9 | 2.3 |
| 7500ppm NaLAS | 7.80E+04 | 4.9 | 2.3 |
| 15ppm KI + 750ppm NaLAS | 4.20E+02 | 2.6 | 4.6 |
| 15ppm KI + 1500ppm NaLAS | 2.00E+02 | 2.3 | 4.9 |

As shown in the table above, the anti-microbial activity of surfactant alone is limited even at high levels of the surfactant. This signifies that the antimicrobial effect of the composition of the present invention cannot be achieved with the individual ingredients alone even at high levels.

### Example 11: Anti-microbial effect of Potassium Iodide and sulphonated Anionic surfactant (Sodium LAS) on Polio virus at pH 10.5

In this example, the improved anti-microbial efficacy of iodide (KI, potassium iodide) and a sulphonated anionic surfactant (LAS, Sodium linear alkylbenzene sulphonate) is demonstrated on a virus (polio virus) at pH 10.5 and compared to the individual components as well as the untreated control sample. The contact time was 15 minutes.

| | N=1 | N=2 | N=3 | Residual log (avg) | Stdev | Log reduction |
|---|---|---|---|---|---|---|
| *Polio virus* control | 5.1 | 4.5 | 4.5 | 4.70 | 0.35 | - |
| 100ppm LAS | 4.1 | 2.9 | 3.3 | 3.43 | 0.61 | 1.27 |
| 20ppm KI | 4.2 | 3.7 | 3.7 | 3.87 | 0.29 | 0.83 |
| 100ppm LAS + 20ppm KI | 2.6 | 2.5 | 1.9 | 2.33 | 0.38 | 2.37 |

As shown in the table above, the individual anti-microbial activity of KI and LAS alone is limited, while the combined efficacy is at least 1-log higher (i.e. 10 fold more reduction).

### Comparative Example 12: Anti-microbial effect of Potassium Iodide and Soap (Laurate) on S.aureus at pH 7

In this example the anti-microbial efficacy of iodide (KI, potassium iodide) and an anionic surfactant (soap) is demonstrated on *S.aureus* at pH 7 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 20 ppm KI | 1.30E+07 | 7.1 | 0.1 |
| 300ppm Laurate | 1.60E+07 | 7.2 | 0.0 |
| 20 ppm KI + 300ppm Laurate | 5.50E+06 | 6.7 | 0.5 |

As shown in the table above, the combined efficacy of KI and Laurate is negligible.

## Claims

1. A detergent composition having anti-microbial benefits comprising:
a 0.5% to 90%w of a sulphonated anionic surfactant wherein the alkyl and acyl groups contain from 8 to 22 carbon atoms ; and
b 0.01 to 0.3%w of an iodine containing inorganic salt selected from potassium iodide and potassium iodate.
wherein the pH of the composition is between 7 and 12.

2. A detergent composition according to claim 1 wherein the composition comprises 3% to 90% of a sulphonated anionic surfactant.

3. A detergent composition according to claim 1 or 2, wherein the sulphonated anionic surfactant is linear alkyl benzene sulfonate (LAS).

4. A composition according to anyone of the preceding claims, wherein the composition further comprises 9% to 94.99%w of one or more of conventional ingredients selected from builder, electrolyte and fillers.

## Patentansprüche

1. Reinigungszusammensetzung mit antimikrobiellen Vorteilen, umfassend:
a 0,5 bis 90 Gew.-% eines sulfonierten anionischen Tensids, worin die Alkyl- und Acyl-Gruppen 8 bis 22 Kohlenstoffatome enthalten, und
b 0,01 bis 0,3 Gew.-% eines Jod-haltigen anorganischen Salzes, das aus Kaliumiodid und Kaliumiodat ausgewählt wird,
wobei der pH der Zusammensetzung zwischen 7 und 12 liegt.

2. Reinigungszusammensetzung nach Anspruch 1, wobei die Zusammensetzung 3 bis 90% eines sulfonierten anionischen Tensids umfasst.

3. Reinigungszusammensetzung nach Anspruch 1 oder 2, wobei das sulfonierte anionische Tensid lineares Alkylbenzolsulfonat (LAS) darstellt.

4. Zusammensetzung nach irgendeinem der vorhergehenden Ansprüche, wobei die Zusammensetzung ferner 9 bis 94,99 Gew.-% eines oder mehrerer herkömmlicher Bestandteile, ausgewählt aus Builder, Elektrolyt und Füllstoffen, umfasst.

## Revendications

1. Composition de détergent présentant des bénéfices antimicrobiens comprenant :
a de 0,5 % à 90 % en masse d'un tensioactif anionique sulfoné où les groupes alkyle et acyle contiennent de 8 à 22 atomes de carbone ; et
b 0,01 à 0,3 % en masse d'un sel inorganique contenant de l'iode choisi parmi de l'iodure de potassium et de l'iodate de potassium.
où le pH de la composition est de 7 à 12.

2. Composition de détergent selon la revendication 1, où la composition comprend de 3 % à 90 % d'un tensioactif anionique sulfoné.

3. Composition de détergent selon la revendication 1 ou 2, où le tensioactif anionique sulfoné est un benzènesulfonate d'alkyle (LAS).

4. Composition selon l'une quelconque des revendications précédentes, où la composition comprend de plus de 9 % à 94,99 % en masse d'un ou plusieurs ingrédients classiques choisis parmi un adjuvant, un électrolyte et des charges.
